(19) Europäisches Patentamt
European Patent Office
Office européen des brevets

(11) Publication number: **0 435 222 A2**

# (12) EUROPEAN PATENT APPLICATION

(21) Application number: 90125274.2

(22) Date of filing: 21.12.90

(51) Int. Cl.[5]: C07D 213/73, C07D 213/75, A61K 31/44

(30) Priority: 27.12.89 US 457610
09.10.90 US 594497

(43) Date of publication of application:
03.07.91 Bulletin 91/27

(84) Designated Contracting States:
AT BE CH DE DK ES FR GB GR IT LI LU NL SE

(71) Applicant: HOECHST-ROUSSEL PHARMACEUTICALS INCORPORATED
Route 202-206 North
Somerville New Jersey 08876(US)

(72) Inventor: Effland, Richard Charles
544 Rolling Hills Road
Bridgewater, NJ08807(US)
Inventor: Klein, Joseph Thomas
1075 Route 202-206
North Bridgewater, NJ 08807(US)

(74) Representative: Becker, Heinrich Karl Engelbert, Dr. et al
HOECHST AKTIENGESELLSCHAFT Central Patent Department P.O. Box 80 03 20
W-6230 Frankfurt am Main 80(DE)

## (54) Aminopyridinylmethanols and aminomethylpyridinamines and related compounds, a process for their preparation and their use as medicaments.

(57) The present invention relates to various compounds of the formula I,

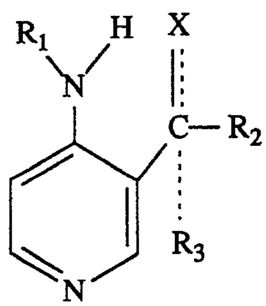

(I),

where
X is OH, O, NOH, $NH_2$, loweralkylamino, cycloalkylamino, arylloweralkylamino or arylamino;
$R_1$ is H, loweralkylcarbonyl, arylcarbonyl, arylloweralkylcarbonyl, loweralkyl or arylloweralkyl;
$R_2$ is hydrogen, loweralkyl, cycloalkyl, arylloweralkyl, aryl; and $R_3$ when present is hydrogen, loweralkyl, cycloalkyl, arylloweralkyl or aryl with the proviso that, if X is OH or $NH_2$, $R_1$, $R_2$ and $R_3$ may not all be hydrogen; and if X = O, $R_2$ may not be hydrogen and $R_1$ may not be 2,2-dimethylpropionyl;
and a process for their preparation.

The compounds of the invention are useful for the treatment of various memory dysfunctions characterized by a cholinergic deficit such as Alzheimer's disease, as topical antiinflammatory agents for the treatment of various dermatoses and also as analgesic agents.

EP 0 435 222 A2

# AMINOPYRIDINYLMETHANOLS AND AMINOMETHYLPYRIDINAMINES AND RELATED COMPOUNDS, A PROCESS FOR THEIR PREPARATION AND THEIR USE AS MEDICAMENTS

The present invention relates to compounds of the Formula I,

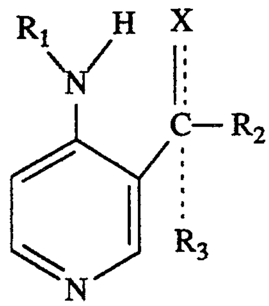

(I)

where
X is OH, O, NOH, $NH_2$, loweralkylamino, cycloalkylamino, arylloweralkylamino or arylamino;
$R_1$ is H, loweralkylcarbonyl, arylcarbonyl, arylloweralkylcarbonyl, loweralkyl or arylloweralkyl;
$R_2$ is hydrogen, loweralkyl, cycloalkyl, arylloweralkyl, aryl; and
$R_3$ when present is hydrogen, loweralkyl, cycloalkyl, arylloweralkyl or aryl with the proviso that if X is OH or $NH_2$, $R_1$, $R_2$ and $R_3$ may not all be hydrogen; and if X = 0, $R_2$ may not be hydrogen and $R_1$ may not be 2,2-dimethylpropionyl;
which compounds are useful for the treatment of various memory dysfunctions characterized by a cholinergic deficit such as Alzheimer's disease, as topical antiinflammatory agents for the treatment of various dermatoses including, for example, exogenous dermatitides (e.g. sunburn, photoallergic dermatitis, urticaria, contact dermatitis, allergic dermatitis), endogenous dermatitides (e.g. atopic dermatitis, seborrheic dermatitis, nummular dermatitis), dermatitides of unknown etiology (e.g. generalized exfoliative dermatitis) and other cutaneous disorders with an inflammatory component (e.g. psoriasis) and also as analgesic agents.

The dotted lines present in Formula (I) and other structural formulas used in this application signify optional bonds. Thus, when the group X is OH, $NH_2$, loweralkylamino, cycloalkylamino, arylloweralkylamino or arylamino, the bond between X and the carbon atom in question is a single bond and a single bond exists between the carbon atom and the group $R_3$, whereas when the group X is O or NOH, the bond between X and the carbon atom is a double bond and the group $R_3$ is non-existent.

Unless otherwise stated or indicated, the following definitions shall apply throughout the specification and the appended claims.

The term loweralkyl shall mean a straight or branched alkyl group having from 1 to 6 carbon atoms. Examples of said loweralkyl include methyl, ethyl, n-propyl, iso-propyl, n-butyl, iso-butyl, sec-butyl, t-butyl and straight- and branched-chain pentyl and hexyl.

The term cycloalkyl shall mean a cycloalkyl group of 3 to 7 carbon atoms.

The term halogen shall mean fluorine, chlorine, bromine or iodine.

The term aryl shall mean a phenyl group optionally mono-substituted with a loweralkyl, loweralkoxy, halogen or trifluoromethyl group.

Throughout the specification and the appended claims, a given chemical formula or name shall encompass all stereo, optical, geometrical and tautomeric isomers where such isomers exist.

The compounds of this invention are prepared by utilizing one or more of the synthetic steps described below.

Throughout the description of the synthetic steps, the notations X, $R_1$, $R_2$ and $R_3$ shall have the respective meanings given above unless otherwise stated or indicated, and other notations shall have the respective meanings defined in their first appearances unless otherwise stated or indicated.

STEP A:

The compound of Formula II (See Turner, J. Org. Chem.,48, 3401-3408 (1983) for its preparation) is allowed to react with a Grignard reagent of the formula $R_2MgBr$ (except that $R_2$ may not he ortho-fluorophenyl) in a routine manner known to the art to afford a compound of Formula III.

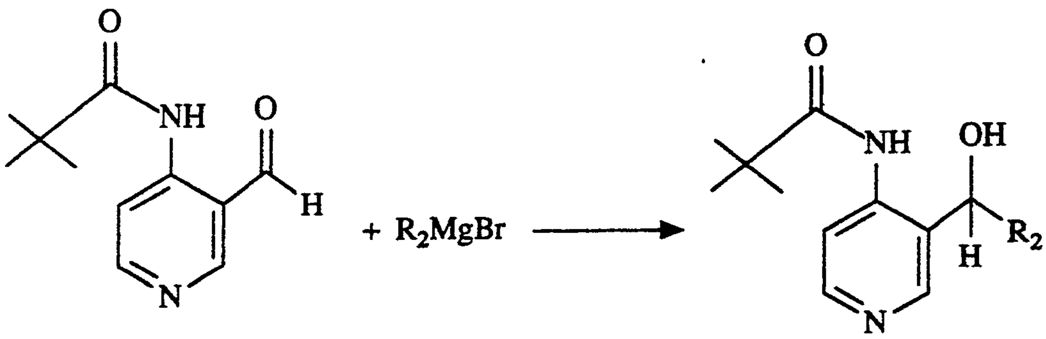


(II) (III)

($-R_2 \neq$ ortho-fluorophenyl)

STEP B

Where the group $-R_2$ is ortho-fluorophenyl, the following alternative route is employed. Thus, 2,2-dimethyl-N-(4-pyridinyl)propanamide is allowed to react with n-BuLi and the resultant dianion is allowed to react with ortho-fluorobenzaldehyde to afford a compound of Formula IV. Typically, the first reaction is conducted in a suitable solvent such as tetrahydrofuran at a temperature of -78 to 25° C and the second reaction is typically conducted in a suitable solvent such as tetrahydrofuran.

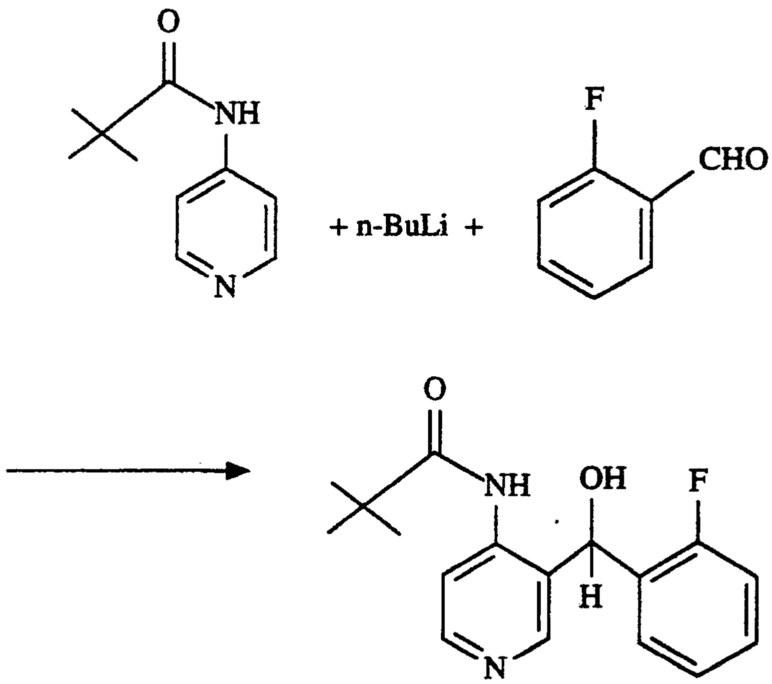


(IV)

In fact, the above reaction is not limited to the preparation of compound IV. Thus, 2,2-dimethyl-N-(4-pyridinyl)propanamide is allowed to react with n-BuLi and the resultant dianion is allowed to react with an aldehyde of the formula $R_2$-CHO to afford a compound of Formula IIIa.

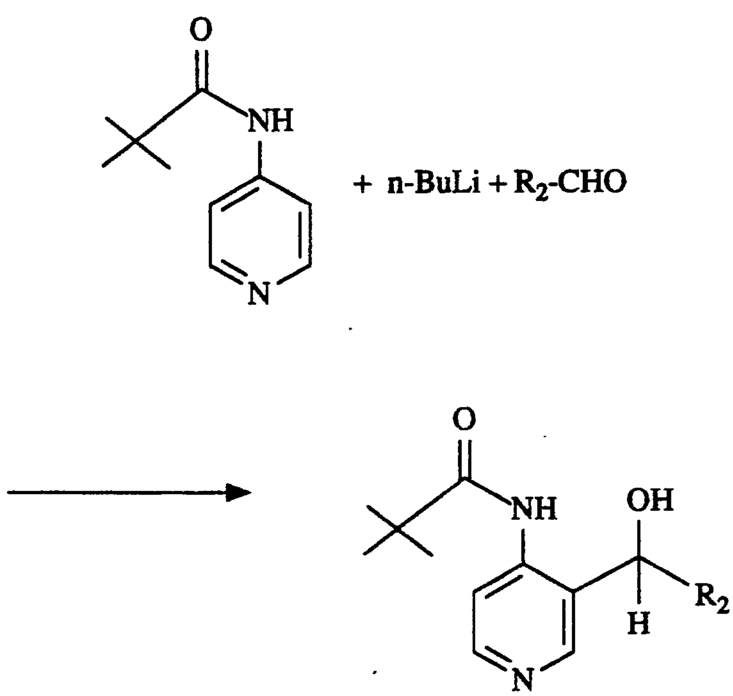


(IIIa)

STEP C:

Compound IIIa which is obtained from STEP A or B is hydrolyzed to afford a compound of Formula V. Said reaction is typically conducted in the presence of NaOH, water and a suitable solvent such as methanol, ethanol or n-propanol, etc. at a temperature of about 20 to 100° C.

(IIIa)

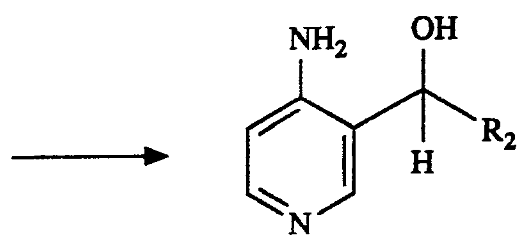

(V)

STEP D:

Compound IIIa is oxidized to afford a compound of Formula VI. Said reaction is typically conducted with the aid of pyridinium dichromate and a suitable solvent such as dimethylformamide or halogenated hydrocarbon at a temperature of about 0 to 150° C.

(IIIa)

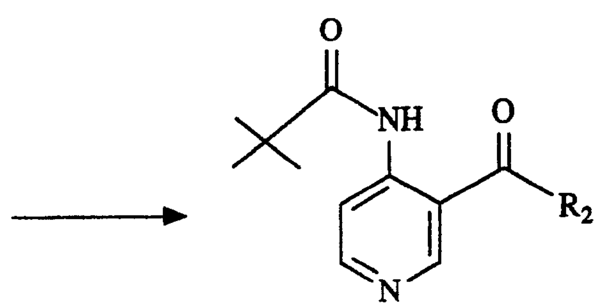

(VI)

STEP E:

Compound VI is hydrolyzed in substantially the same manner as in STEP C to afford a compound of Formula VII.

(VI) 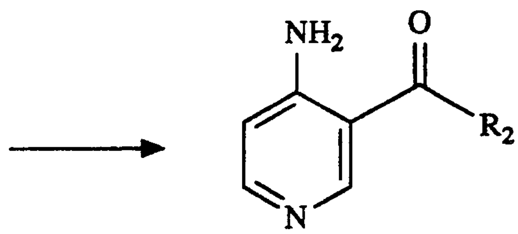

(VII)

STEP F

Compound VII is allowed to react with an acid chloride of the formula $R'_1$-CO-Cl where $R'_1$ is loweralkyl, arylloweralkyl or aryl in a routine manner known to the art to afford a compound of Formula VIII.

(VII) + $R'_1$-CO-Cl 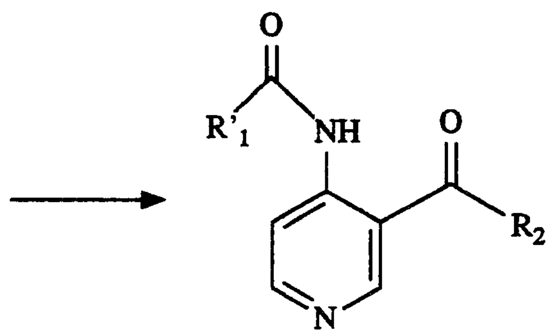

(VIII)

STEP G

Compound VI is allowed to react with a primary amine of the formula $H_2N$-$R_4$ where $R_4$ is loweralkyl, cycloalkyl, arylloweralkyl or aryl and the resultant imine of Formula IX is reduced with $NaBH_4$ to afford a compound of Formula X.

The first reaction is typically conducted in the presence of an acid such as p-toluenesulfonic acid and a suitable solvent such as benzene, toluene or xylene at a temperature of about 80 to 150 °C. The second reaction is typically conducted in a suitable solvent such as isopropanol, ethanol or methanol at a temperature of about 0 to 80 °C.

(VI) + 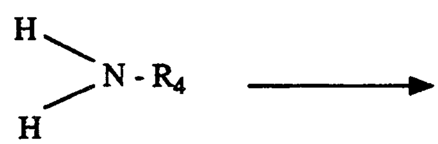

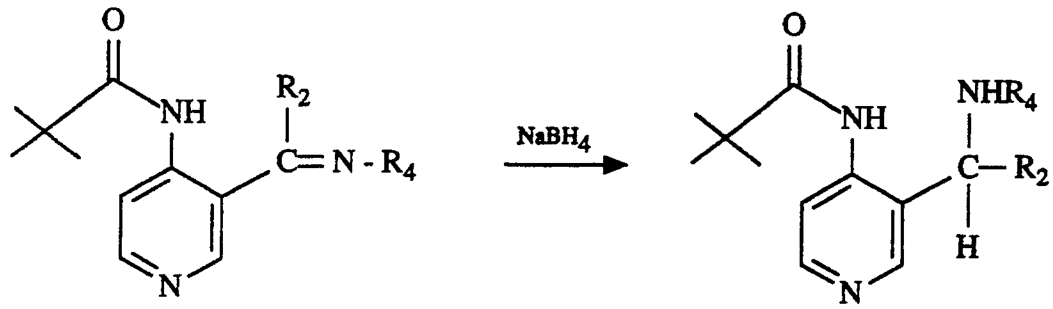


(IX) (X)

STEP H

Compound IX is allowed to react with a Grignard reagent of the formula $R_3MgBr$ or $R_3MgCl$ ($R_3 \neq H$) in a routine manner known to the art to afford a compound of Formula XI.

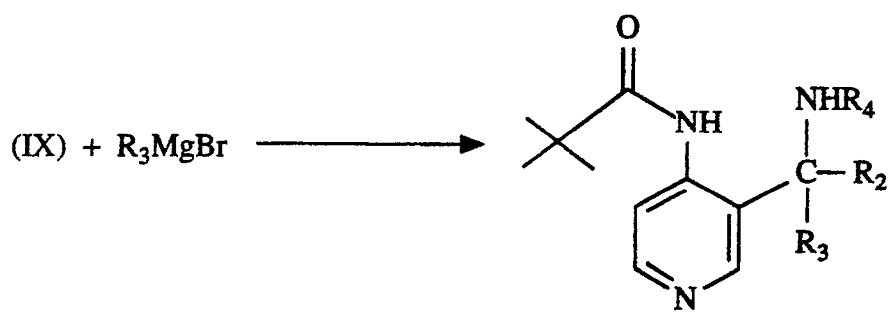


(XI)

($R_3 \neq H$)

STEP I

Compound X or XI is hydrolyzed in substantially the same manner as in STEP C to afford a compound of Formula XII.

(X) or (XI)

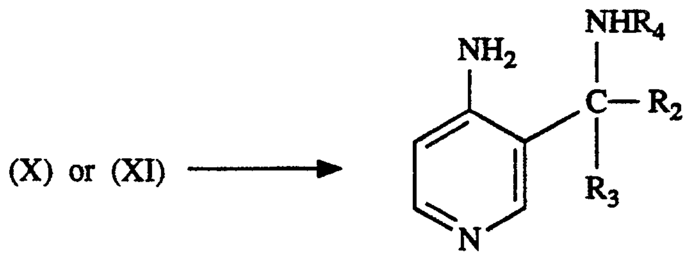

(XII)

STEP J

Using Compound VIII as a starting compound, STEP G is repeated in substantially the same manner to afford compounds of Formula XIII and XIV.

(VIII)

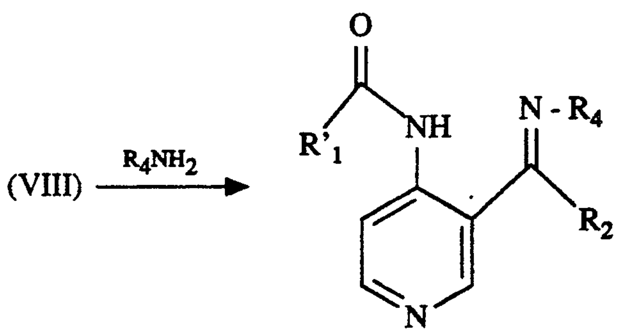


(XIII)

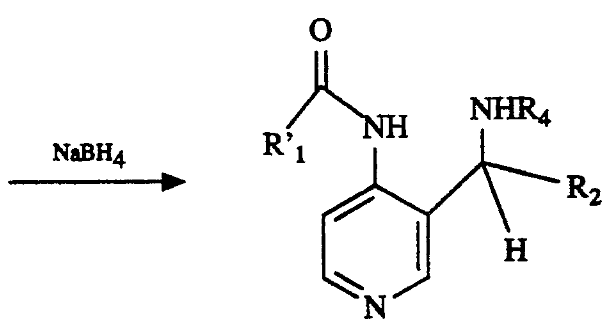


(XIV)

STEP K

Compound XIV is reduced with $LiAlH_4$ in a routine manner known to the art to afford a compound of Formula XV.

(XIV)

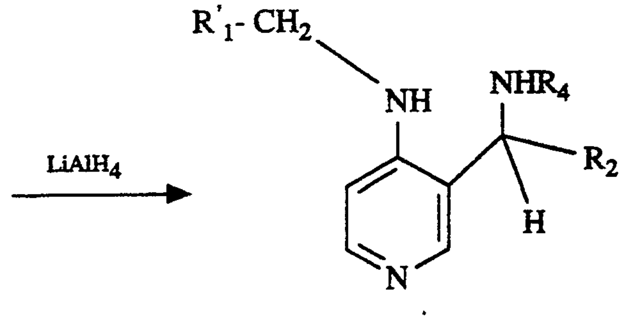


(XV)

STEP L

Compound VII is allowed to react with hydroxylamine hydrochloride to afford an oxime compound of Formula XVI. This reaction is typically conducted in a suitable solvent such as pyridine at a temperature of about 20 to 120 ° C.

(VII) + $NH_2OH \bullet HCl$

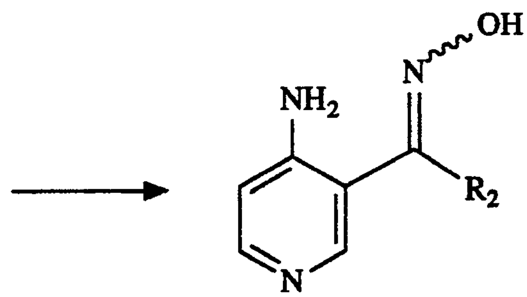

(XVI)

STEP M

Compound XVI is hydrogenated with the aid of a Raney alloy to afford a compound of Formula XVII. This reaction is typically conducted by adding a Raney alloy (for instance a 50:50 Al/Ni alloy) to a solution of compound XVI in a suitable solvent such as ethanol and thereafter adding an aqueous solution of NaOH to the reaction mixture. The reaction is typically conducted at a temperature of about 0 to 80 ° C.

(XVI) + $H_2$

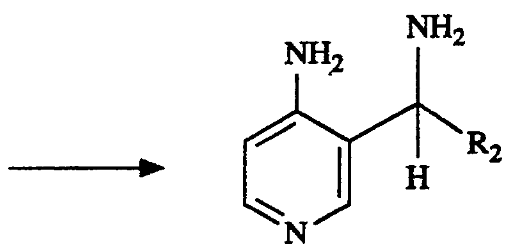

(XVII)

Compounds of Formula I according to this invention are useful as topical agents for the treatment of

various skin disorders such as those mentioned earlier. The dermatological activities of the compounds of this invention were ascertained with reference to the following methods.

DERMATOLOGICAL TEST METHODS

Phospholipase $A_2$-induced Paw Edema (PIPE)

The ability of compounds to prevent naja naja (snake venom) phospholipase $A_2$-induced paw edema in male Wistar rats (100-125 g) was measured. $PLA_2$ (3 units/paw) alone or with 0.1 M of the test compound was injected in the subplantar region of the rat left hindpaw. Immediately subsequent to the injection and at two hours post administration the paw was immersed in a mercury bath, and paw displacement was measured on a recorder via a transducer (Standard: hydrocortisone $ED_{50}$ = 0.46 M). See Giessler, A.J. et al., ***Agents and Actions***, Vol. 10, Trends in Inflammation Research (1981), p. 195.

In Vitro Phospholipase $A_2$ Assay ($PLA_2$)

The ability of a compound to modulate $PLA_2$ activity (cleavage of $^{14}C$-dipalmitoyl phosphotidylcholine at the 2-position to $^{14}C$-palmitic acid) was quantitated in this assay. The reaction mixture contained Tris buffer (25mM), pH 8.0, calcium chloride (2.0 mM), bovine serum albumin (0.5 mg), dipalmitoyl phosphotidylcholine ($8x10^{-5}$M), ($^{14}C$-palmitoyl)dipalmitoyl phosphotidylcholine ($6x10^3$ cpm), porcine pancreatic $PLA_2$ (3.2 units) and the test compound. The reaction was run at 37 ° C in a shaking incubator. The reaction was quenched and an internal standard was added in order to determine sample recovery. The samples were loaded onto $C_{18}$ columns, eluted with ethanol, and the radioactivity was then measured (Standard: quinacrine $IC_{50}$ = $3.5x10^{-4}$M). See Feyen, J.H.M., et al., **Journal of Chromatography** 259 (1983), pp. 338-340.

Arachidonic Acid-Induced Ear Edema (AAEE)

The purpose of this assay was to determine the ability of a topically-applied compound to prevent mouse ear edema induced by topical application of arachidonic acid. Female Swiss Webster mice topically received vehicle or test compound (1.0 mg/ear) on both ears (10µl on outer and inner ears). After 30 minutes, the right ear of all groups received arachidonic acid (4 mg/ear) and the left ear received vehicle alone. After an additional 1 hour, the mice were sacrificed and an ear punch (4 mm) was taken from each ear. The difference in right and left ear punch weights for each animal was determined to assess activity (Standard: indomethacin $ED_{50}$ = 1.5 mg/ear). See Young, J.M. et al., ***J. Invest. Dermatol.***, 80, (1983), pp 48-52.

TPA-Induced Ear Edema (TPAEE)

The purpose of this assay was to determine the ability of a topically-applied compound to prevent ear edema induced by topical application of TPA (phorbol 12-myristate acetate). Female Swiss Webster mice topically received TPA (10µg/ear) on the right ear and vehicle on the left ear. The test compound (10 µg/ear) was applied to both ears. After five hours, the animals were sacrificed and an ear punch (4 mm) was taken from each ear. The difference in right and left ear punch weights for each animal was determined to assess activity (Standard: hydrocortisone $ED_{50}$ = 47 µg/ear). See Young, J.M. et al., ***J. Invest. Dermatol.***, 80 (1983), pp. 48-52.

Dermatological activities for some of the compounds of this invention are presented in Table 1.

| TABLE 1 | | | | |
|---|---|---|---|---|
| Compound | PIPE* (0.1 M) | $PLA_2$* (0.01 M) | AAEE (1 mg) | TPAEE (10 µg) |
| α-cyclohexyl-4-(2,2-dimethyl-propionamido)-3-pyridinylmethanol | | | -52% | -55% |
| α-phenyl-4-amino-3-pyridinyl-methanol hydrochloride | | -87% | | |
| α-(2-fluoro-phenyl)-4-amino-3-pyridinylmethanol hydrochloride | -56% | | | |
| (4-amino-3-pyridinyl)cyclo-hexylmethanone hydrochloride | | -82% | | -53% |
| N-[3-[1-(phenylmethyl-amino)ethyl]-4-pyridinyl]-2,2-dimethylpropanamide dihydrochloride | | -74% | | |
| 3-[(phenyl-methylamino)-methyl]-4-pyridinamine dihydrochloride | -51% | -79% | | |
| 4-amino-α-methyl-N-(phenylmethyl)-3-pyridinemethanamine | -44% | | | |
| (4-amino-3-pyridinyl)cyclo-hexylmethanone oxime hydrochloride | | -99% | | |
| (4-amino-3-pyridinyl)-(2-fluorophenyl)-methanone oxime | | -32% | | -56% |

| | | |
|---|---|---|
| (4-amino-3-pyridinyl)cyclohexylmethanamine dihydrochloride | -68% | -72% |
| (4-amino-3-pyridinyl)-benzenemethanamine dihydrochloride | -66% | |

* difference in edema vs. control

The compounds of formula I of the present invention are also useful for the treatment of various memory dysfunctions characterized by a decreased cholinergic function such as Alzheimer's disease.

This utility is demonstrated by the ability of these compounds to restore cholinergically deficient memory in the Dark Avoidance Assay.

Dark Avoidance Assay

In this assay mice are tested for their ability to remember an unpleasant stimulus for a period of 24 hours. A mouse is placed in a chamber that contains a dark compartment: a strong incandescent light drives it to the dark compartment, where an electric shock is administered through metal plates on the floor. The animal is removed from the testing apparatus and tested again, 24 hours later, for the ability to remember the electric shock.

If scopolamine, an anticholinergic that is known to cause memory impairment, is administered before an animal's initial exposure to the test chamber, the animal re-enters the dark compartment shortly after being placed in the test chamber 24 hours later. This effect of scopolamine is blocked by an active test compound, resulting in a greater interval before re-entry into the dark compartment.

The results for an active compound are expressed as the percent of a group of animals in which the effect of scopolamine is blocked, as manifested by an increased interval between being placed in the test chamber and re-entering the dark compartment.

Results of this assay for some of the compounds of this invention and physostigmine (reference compound) are presented in Table 2.

TABLE 2

| Compound | Dose (mg/kg of body weight, s.c) | % of Animals with Scopolamine Induced Memory Deficit Reversal |
|---|---|---|
| α-methyl-4-amino-3-pyridinylmethanol hydrochloride | 1.25 | 33% |
| α-cyclohexyl-4-(2,2-dimethyl-propionamido)-3-pyridinylmethanol | 0.16 | 33% |
| α-cyclohexyl-4-amino-3-pyridinyl-methanol hydrochloride | 0.16 | 50% |
| α-phenyl-4-(2,2-dimethyl-propionamido)-3-pyridinylmethanol | 0.31 | 40% |
| α-phenyl-4-amino-3-pyridinyl-methanol hydrochloride | 0.63 | 27% |
| 3-(butylamino)-methyl-4-pyridinamine dihydrochloride | 0.30 | 27% |
| α-(2-fluoro-phenyl)-4-amino-3-pyridinylmethanol hydrochloride | 1.0 | 40% |
| (4-amino-3-pyridinyl)-benzenemethanamine dihydrochloride | 0.30 | 27% |
| (Reference Compound) | | |
| Physostigmine | 0.31 | 20% |

Compounds I of the present invention are also useful as analgesic agents due to their ability to alleviate pain in mammals. The activity of the compounds is demonstrated in the 2-phenyl-1,4-benzoquinone-induced writhing (PQW) test in mice, a standard assay for analgesics [Proc. Soc. Exptl. Biol. Med., 95, 729 (1957)].

Inhibition of Phenylquinone-Induced Writhing in Mice (PQW)

A 0.125% concentration of phenyl-p-benzoquinone in a 5% aqueous solution of ethyl alcohol is administered to mice (10 mL/kg, ip). This produces a characteristic "writhe" which is defined as an inward rotation of one or more feet with twisting and turning of the trunk, drawing in of the abdominal wall, lordosis, and arching of the back. A total of 28 male CD-1 Charles River mice (18-30 g) are employed for a time-response. Animals receive food and water ad libitum during their stay in the animal quarters prior to testing. Compounds are tested at 20 mg/kg, sc and are prepared with distilled water, and if insoluble one drop of Tween-80, a surfactant is added. Compounds are administered in a dosage volume of 10 mL/kg.

Twenty mice (five per group) are administered the test compound at various pretreat time (e.g., 15,30,45, and 60 min) prior to phenylquinone injection. Control animals (two per group) receive an equal volume of vehicle. After the administration of phenylquinone, the mice are placed separately into 1-L beakers, and 5 min are allowed to elapse. The mice are then observed for a period of 10 min, and the number of writhes is recorded for each animal. The formula for computing percent inhibition is

$$\frac{(\bar{X}\text{ writhes in control group}) - (\bar{X}\text{ writhes in drug group})}{\bar{X}\text{ writhes in control group}} \times 100\%$$

The time period with the maximum percent of inhibition is considered the peak time. A dose-response is reserved for interesting compounds or those which inhibit writhing by 70% or more. A dose-response is run in the same manner as a time-response except 10 animals per group are tested at the peak time of drug activity. Fifty animals, divided among four drug groups and one vehicle control group, are employed. The mice are normally given four doses of drug, each twice the amount of the preceding dose. An $ED_{50}$ is calculated by a computer linear regression analysis.

Results of this assay for some of the compounds of this invention a reference compound are presented in Table 3.

## TABLE 3

## ANALGESIC ACTIVITY

| Compound | PQW (% inhibition of writhing) | Dose (mg/kg, s.c.) |
|---|---|---|
| α-methyl-4-(2,2-dimethylpropionamido)-3-pyridinylmethanol | 89% | 20 |
| α-phenyl-4-(2,2-dimethylpropionamido)-3-pyridinylmethanol | 38% | 20 |
| 3-(butylamino)methyl-4-pyridinamine dihydrochloride | 62% | 20 |
| 3-[(phenylmethylamino)-methyl]-4-pyridinamine dihydrochloride | 40% | 20 |
| (Reference Compound) Propoxyphene | 50% | 3.9 |

Effective quantities of the compounds of the invention may be administered to a patient by any of the various methods, for example, orally as in capsule or tablets, parenterally in the form of sterile solutions or suspensions, and in some cases intravenously in the form of sterile solutions. The free base final products, while effective themselves, may be formulated and administered in the form of their pharmaceutically acceptable acid addition salts for purposes of stability, convenience of crystallization, increased solubility and the like.

Acids useful for preparing the pharmaceutically acceptable acid addition salts of the invention include inorganic acids such as hydrochloric, hydrobromic, sulfuric, nitric, phosphoric and perchloric acids, as well as organic acids such as tartaric, citric, acetic, succinic, maleic, fumaric and oxalic acids.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compounds, but may be varied depending upon the particular form and may conveniently be between 4% to about 70% of the weight of the unit. The amount of active compound in such compositions is such that a suitable dosage will be obtained. Preferred compositions and preparations according to the present invention are prepared so that an oral dosage unit form contains between 1.0 - 300 milligrams of active compound.

The tablets, pills, capsules, troches and the like may also contain the following ingredients: a binder such as micro-crystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxide; and a sweetening agent such as sucrose or saccharin may be added or a flavoring agent such as peppermint, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes, coloring and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0.1% of active compound, but may be varied between 0.5 and about 30% of the weight thereof. The amount of active compound in such compositions is such that a suitable dosage will be obtained. Preferred compositions and preparations according to the present inventions are prepared so that a parenteral dosage unit contains between 0.5 to 100 milligrams of active compound.

Examples of the compounds of this invention include:
α-methyl-4-(2,2-dimethylpropionamido)-3-pyridinylmethanol;
α-cyclohexyl-4-(2,2-dimethylpropionamido)-3-pyridinylmethanol;
α-phenyl-4-(2,2-dimethylpropionamido)-3-pyridinylmethanol;
α-(2-fluorophenyl)-4-(2,2-dimethylpropionamido)-3-pyridinylmethanol;
α-(2-fluorophenylmethyl)-4-(2,2-dimethylpropionamido)-3-pyridinyl-methanol;
α-methyl-4-amino-3-pyridinylmethanol;
α-cyclohexyl-4-amino-3-pyridinylmethanol;
α-phenyl-4-amino-3-pyridinylmethanol;
α-(2-fluorophenyl)-4-amino-3-pyridinylmethanol;
N-(3-acetyl-4-pyridinyl)-2,2-dimethylpropanamide;
N-[3-(cyclohexylcarbonyl)-4-pyridinyl]-2,2-dimethylpropanamide;
(4-amino-3-pyridinyl)cyclohexylmethanone;
N-[3-[(butylamino)methyl]A-pyridinyl]-2,2-dimethylpropanamide;
N-[3-[(phenylmethylamino)methyl]-4-pyridinyl]-2,2-dimethylpropanamide;
N-[3-[1-(phenylmethylamino)ethyl]-4-pyridinyl]-2,2-dimethylpropanamide;
3-(butylamino)methyl-4-pyridinamine;
3-[(phenylmethylamino)methyl]-4-pyridinamine;
4-amino-α-methyl-N-(phenylmethyl)-3-pyridinemethanamine;
(4-amino-3-pyridinyl)cyclohexylmethanone oxime;
(4-amino-3-pyridinyl)phenylmethanone oxime;
(4-amino-3-pyridinyl)-(2-fluorophenyl)-methanone oxime;
(4-amino-3-pyridinyl)cyclohexylmethanamine;
(4-amino-3-pyridinyl)benzenemethanamine;
α-ethyl-4-aminopyridine-3-methanol;
α-phenylmethyl-4-aminopyridine-3-methanol;
α-methyl-4-phenylmethylamino-3-pyridinemethanamine;
α-phenylmethyl-4-amino-3-pyridinemethanamine;
(4-propylamino-3-pyridinyl)benzenemethanamine;
α-(3,4-dimethoxyphenylmethyl)-4-amino-3-pyridinemethanamine;

The following examples are presented in order to illustrate this invention. All temperatures are given in degrees Celcius.

## EXAMPLE 1

### α-Methyl-4-(2,2-dimethylpropionamido)-3-pyridinylmethanol

To an ice-cooled solution of N-(3-formyl-4-pyridinyl)-2,2-dimethylpropanamide[1] (8 g) in 150 ml tetrahydrofuran was added methylmagnesium bromide (3.0 M in ether, 30 ml). After thirty minutes the reaction mixture was stirred with a saturated ammonium chloride solution, basified with sodium carbonate and extracted with ether. The organic extract was washed with water and a saturated sodium chloride solution, dried over anhydrous magnesium sulfate, filtered and evaporated to an oil. This oil was purified by flash chromatography (silica, ethyl acetate) to give 7 g oil. This oil was crystallized from ether-hexane to give 4.5 g solid, mp 100-102°. This solid was recrystallized from ether-hexane to give 4.0 g solid, mp 100-102°.

[1]Turner, J. Org. Chem. 48, 3401-3408 (1983)

ANALYSIS:

| | | | |
|---|---|---|---|
| Calculated for $C_{12}H_{18}N_2O_2$: | 64.84%C | 8.16%H | 12.61%N |
| Found: | 64.82%C | 8.23%H | 12.58%N |

**EXAMPLE 2**

**α-Cyclohexyl-4-(2,2-dimethylpropionamido)-3-pyridinylmethanol**

To an ice-cooled solution of N-(3-formyl-4-pyridinyl)-2,2-dimethylpropanamide (10 g) in 100 ml tetrahydrofuran was added cyclohexylmagnesium chloride (2.0 M in ether, 35 ml). After thirty minutes the reaction mixture was stirred with a saturated ammonium chloride solution, basified with sodium carbonate and extracted with ether. The organic extract was washed with water and a saturated sodium chloride solution, dried over anhydrous magnesium sulfate, filtered and evaporated to an oil. This oil was purified by flash chromatography (silica, 20% ethyl acetate in dichloromethane, then ethyl acetate) to give 6 g oil. This oil was crystallized from hexane to give 2.5 g solid, mp 148-150°. This solid was recrystallized from acetonitrile to give 2.1 g crystals, mp 153-155°.

ANALYSIS:

| | | | |
|---|---|---|---|
| Calculated for $C_{17}H_{26}N_2O_2$: | 70.31%C | 9.02%H | 9.65%N |
| Found: | 70.11%C | 9.08%H | 9.65%N |

**EXAMPLE 3**

**α.Phenyl-4-(2,2-dimethylpropionamido)-3-pyridinylmethanol**

To an ice-cooled solution of N-(3-formyl-4-pyridinyl)-2,2-dimethylpropanamide (15 g) in 150 ml tetrahydrofuran was added phenylmagnesium chloride (2.0 M in tetrahydrofuran, 75 ml). After one hour the reaction mixture was stirred with a saturated ammonium chloride, basified with sodium carbonate and extracted with ether. The organic extract was washed with water and a saturated sodium chloride solution, dried over anhydrous magnesium sulfate, filtered and evaporated to a waxy solid. This solid was purified by flash chromatography (silica, 20% ethyl acetate in dichloromethane, then ethyl acetate) to give 16 g solid. A four gram portion was recrystallized from acetonitrile to give 3.2 g solid, mp 151-153°. This solid was recrystallized from acetonitrile to give 2.7 g crystals, mp 154-155°.

ANALYSIS:

| | | | |
|---|---|---|---|
| Calculated for $C_{17}H_{20}N_2O_2$: | 71.80%C | 7.09%H | 9.85%N |
| Found: | 71.85%C | 7.14%H | 9.95%N |

**EXAMPLE 4**

**α-(2-Fluorophenyl)-4-(2,2-dimethylpropionamido)-3-pyridinylmethanol**

To a cooled solution of 2,2-dimethyl-N-(4-pyridinyl)propanamide (15g) in 250 ml tetrahydrofuran was slowly added n-butyllithium (2.5 M in hexane, 108 ml) at such a rate that the internal temperature was

maintained between -10° and -5°. After one hour a solution of 2-fluorobenzaldehyde (21 g) in 25 ml tetrahydrofuran was slowly added. After one hour the reaction mixture was stirred with water and extracted with ether. The organic extract was washed with water and a saturated sodium chloride solution, dried (anhy. $MgSO_4$), filtered and evaporated to 30 g of an oil. This was purified by flash chromatography (silica, 20% ethyl acetate in dichloromethane) to yield 11 g of the product as a solid, mp 178-180°. Four grams were recrystallized from acetonitrile to yield 3.7 g crystals, mp 180-182°.

ANALYSIS:

| | | | |
|---|---|---|---|
| Calculated for $C_{17}H_{19}FN_2O_2$: | 67.53%C | 6.33%H | 9.27%N |
| Found: | 67.36%C | 6.31%H | 9.24%N |

**EXAMPLE 5**

**α-(2-Fluorophenylmethyl)-4-(2,2-dimethylpropionamido)-3-pyridinylmethanol maleate**

A solution of 2-fluorobenzyl chloride (30.4 g) in 150 ml anhydrous ether was added to magnesium shavings (4.1 g) at such a rate that maintained ether reflux. (Grignard formation was initiated in situ with 1,2-dibromoethane). The freshly prepared Grignard reagent was cooled with an ice bath and a solution of 4-(2,2-dimethylpropionamido)-pyridine-3-carboxaldehyde (17.4 g) in 150 ml ether was added at a rate such that the internal temperature was maintained below 7°. After one hour the mixture was quenched by slow addition of 250 ml cold water and thereafter basified with sodium carbonate and separated. The aqueous layer was extracted with ether and the combined organics were washed with water and a saturated sodium chloride solution. The dried (anhydrous magnesium sulfate) organic layer was filtered and evaporated to 41 g oil. This oil was eluted through silica with 20% ethyl acetate in dichloromethane to give 18.3 g oil. An 8.3 g portion was converted to the maleate salt in methanol-ether to yield 5 g solid, d 146-148°. A 2 g portion was recrystallized from methanol-ether to yield 1.5 g crystals, d 154-155°.

ANALYSIS:

| | | | |
|---|---|---|---|
| Calculated for $C_{22}H_{25}FN_2O_6$: | 61.10%C | 5.83%H | 6.48%N |
| Found: | 61.05%C | 5.82%H | 6.41%N |

**EXAMPLE 6**

**α-Methyl-4-amino-3-pyridinylmethanol hydrochloride**

A solution prepared from α-methyl-4-(2,2-dimethylpropionamido)-3-pyridinylmethanol (6.2 g) in 150 ml methanol and 15 ml 10% aqueous sodium hydroxide was allowed to stand two days at ambient temperature and thereafter evaporated. The resultant residue was purified by flash chromatography (silica, 25% methanol in dichloromethane) to give 3 g solid, mp 118-120°. This was combined with 2 g product obtained from a previous hydrolysis and purified by HPLC (silica, 25% methanol in dichloromethane) to give 3.5 g oil. This oil was converted to the hydrochloride salt in methanol-ether to give 2.7 g solid, mp 198-200°. This was recrystallized from methanol-ether to give 2.5 g solid, mp 198-200°.

ANALYSIS:

| | | | |
|---|---|---|---|
| Calculated for $C_7H_{10}N_2O{\bullet}HCl$: | 48.15%C | 6.35%H | 16.04%N |
| Found: | 47.73%C | 6.34%H | 15.82%N |

**EXAMPLE 7**

**α-Cyclohexyl-4-amino-3-pyridinylmethanol hydrochloride**

A solution prepared from α-cyclohexyl-4-(2,2-dimethylpropionamido)-3-pyridinylmethanol (7 g) in 150 ml methanol and 15 ml 10% aqueous sodium hydroxide was stirred at reflux for twelve hours and thereafter cooled and evaporated. The resultant residue was purified by flash chromatography (silica, 25% methanol in dichloromethane) to give 6 g oil. This oil was crystallized in ether to give 3.6 g solid, mp 140-142°. This solid was converted to the hydrochloride salt in methanol-ether to give 2.1 g crystals, d 220-222°.

ANALYSIS:

| | | | |
|---|---|---|---|
| Calculated for $C_{12}H_{18}N_2O{\bullet}HCl$: | 59.37%C | 7.89%H | 11.54%N |
| Found: | 59.12%C | 7.92%H | 11.28%N |

**EXAMPLE 8**

**α.Phenyl-4-amino-3-pyridinylmethanol hydrochloride**

A solution prepared from α-phenyl-4-(2,2-dimethylpropionamido)-3-pyridinylmethanol (12 g) in 250 ml methanol and 25 ml 10% aqueous sodium hydroxide was warmed on a steam bath for one hour and thereafter cooled, evaporated and purified by flash chromatography (silica, 20% methanol in dichloromethane) to give 8 g oil. This oil was converted to the hydrochloride salt in methanol-ether to give 5 g solid, mp 198-200°. Three grains were recrystallized from methanol-ether to give 2.7 g crystals, mp 201-202°.

ANALYSIS:

| | | | |
|---|---|---|---|
| Calculated for $C_{12}H_{12}N_2O{\bullet}HCl$: | 60.89%C | 5.54%H | 11.84%N |
| Found: | 60.84%C | 5.60%H | 11.76%N |

**EXAMPLE 9**

**α-(2-Fluorophenyl)-4-amino-3-pyridinylmethanol hydrochloride**

A solution prepared from α-(2-fluorophenyl)-4-(2,2-dimethylpropionamido)-3-pyridinylmethanol (18 g) in 200 ml methanol and 20 ml 10% aqueous sodium hydroxide was stirred at 75-80° for two hours and thereafter cooled, evaporated, stirred with water and extracted with ethyl acetate-ether. The organic extract was washed with water and saturated sodium chloride and thereafter dried (anhy. $MgSO_4$), filtered and evaporated to 13 g of an oil. This was purified by flash column chromatography (silica, 20% methanol in dichloromethane) to yield 11.5 g of the product as a glassy solid, mp 60-65°. Three grams were converted to the hydrochloride salt in methanol-ether to yield 2.8 g solid, mp 210-213°. This was recrystallized from methanol-ether to yield 2.2 g crystals, mp 215-216°.

ANALYSIS:

| | | | |
|---|---|---|---|
| Calculated for $C_{12}H_{12}ClFN_2O$: | 56.59%C | 4.75%H | 11.00%N |
| Found: | 56.60%C | 4.75%H | 10.94%N |

## EXAMPLE 10

### N-(3-Acetyl-4-pyridinyl)-2,2-dimethylpropanamide

A solution of α-methyl-4-(2,2-dimethylpropionamido)-3-pyridinylmethanol (11 g) and pyridinium dichromate (26 g) in 100 ml dimethylformamide was stirred at ambient temperature for twenty hours and thereafter stirred with water, basified with sodium carbonate and extracted with ether. The organic extract was washed with water and a saturated sodium chloride solution, dried (anhy. $MgSO_4$), filtered and evaporated to 11 g solid. This solid was purified by flash chromatography (silica, 20% ethyl acetate in dichloromethane) to yield 7 g solid, mp 95°. Six grams were recrystallized from hexane to yield 4.8 g crystals, mp 98-100°. An analytical sample was obtained by recrystallizing 3 g from hexane to yield 2.7 g crystals, mp 98-100°.

ANALYSIS:

| | | | |
|---|---|---|---|
| Calculated for $C_{12}H_{16}N_2O_2$: | 65.43%C | 7.32%H | 12.72%N |
| Found: | 65.67%C | 7.52%H | 12.53%N |

## EXAMPLE 11

### N-[3-(Cyclohexylcarbonyl)-4-pyridinyl]-2,2-dimethylpropanamide hydrochloride

Pyridinium dichromate (30 g) was added to a solution of α-cyclohexyl-4-(2,2-dimethylpropionamido)-3-pyridinylmethanol (16 g) in 150 ml dimethylformamide and the resultant solution was stirred for sixteen hours at ambient temperature, and thereafter stirred with water, basified with sodium carbonate and extracted with ethyl acetate-ether. The organic extract was washed with water and a saturated sodium chloride solution, dried (anhy. $MgSO_4$), filtered and evaporated to 12 g solid. This was purified by flash chromatography (silica, 10% ethyl acetate in dichloromethane) to yield 11 g solid. This was purified by column chromatography (alumina, ether) to yield 9 g solid, mp 80°. Three grams were converted to the hydrochloride salt in ether to yield 3 g solid, mp 212-215°. This was recrystallized from methanol-ether (1:40) to yield 2.2 g crystals, mp 216-218°.

ANALYSIS:

| | | | |
|---|---|---|---|
| Calculated for $C_{17}H_{25}ClN_2O_2$: | 62.85%C | 7.76%H | 8.63%N |
| Found: | 63.25%C | 7.67%H | 8.61%N |

## EXAMPLE 12

### (4-Amino-3-pyridinyl)cyclohexylmethanone hydrochloride

A solution prepared from N-[3-(cyclohexylcarbonyl)-4-pyridinyl]-2,2-dimethylpropanamide (5.5g) in 100 ml methanol and 10 ml 10% aqueous sodium hydroxide was stirred for three hours at ambient temperature and thereafter evaporated, stirred with water and extracted with ethyl acetate. The organic extract was washed with water and a saturated sodium chloride solution, dried (anhy. $MgSO_4$), filtered and evaporated to 5 g oil. This oil was purified by flash chromatography (silica, ethyl acetate) to yield 4 g viscous oil. This was converted to the hydrochloride salt in methanol-ether to yield 4.2 g crystals, dec. 266-270°.

ANALYSIS:

| | | | |
|---|---|---|---|
| Calculated for $C_{12}H_{17}ClN_2O$: | 59.87%C | 7.12%H | 11.64%N |
| Found: | 59.80%C | 7.07%H | 11.56%N |

EXAMPLE 13

N-[3-[(Butylamino)methyl]-4-pyridinyl]-2,2-dimethylpropanamide dihydrochloride

A solution of N-(3-formyl-4-pyridinyl)-2,2-dimethylpropanamide (10 g), n-butylamine (7.1 g) and p-toluenesulfonic acid monohydrate (0.1 g) in 150 ml toluene was stirred at reflux with removal of water. After six hours the solution was cooled and evaporated and the product was purified by flash chromatography (silica, 50% ethyl acetate in dichloromethane) to yield 8 g oil. The IR ($CHCl_3$), NMR ($CDCl_3$) and mass spectra ($MH^+$ = 262) were consistent with the structure of the imine intermediate. A solution of the imine (combined with 3 g obtained from another condensation) in 100 ml isopropanol and 25 ml methanol was stirred for one hour at 80° with sodium borohydride (5 g), and thereafter cooled, stirred with water and extracted with ether. The organic extract was washed with water and a saturated sodium chloride solution, dried (anhy. $MgSO_4$), filtered and evaporated to an oil. This oil was purified by flash chromatography (silica, 50% ethyl acetate in dichloromethane) to yield 6.5 g oil. This oil was purified by column chromatography (alumina, ether) to yield 6 g oil. An analytical sample was obtained by converting 2.5 g to the dihydrochloride salt in methanol-ether to yield 2.7 g crystals, mp 166-168°.

ANALYSIS:

| | | | |
|---|---|---|---|
| Calculated for $C_{15}H_{25}N_3O{\bullet}2HCl$: | 53.57%C | 8.09%H | 12.50%N |
| Found: | 53.58%C | 8.09%H | 12.53%N |

EXAMPLE 14

N-[3-[(Phenylmethylamino)methyl]-4-pyridinyl]-2,2-dimethylpropanamide dihydrochloride

A solution of N-(3-formyl-4-pyridinyl)-2,2-dimethylpropanamide (10g), benzylamine (8 g) and p-toluenesulfonic acid monohydrate (0.1 g) in 125 ml toluene was stirred at reflux with removal of water. After two hours the solution was cooled and evaporated and the product was purified by flash chromatography (silica, 20% ethyl acetate in dichloromethane) to yield 11.3 g solid, mp 105-109°. The IR ($CHCl_3$), NMR ($CDCl_3$) and mass spectra were consistent with the structure of the imine intermediate. A solution of the imine (11 g) in 100 ml isopropanol and 25 ml methanol was stirred for one hour at 80° with sodium borohydride (5 g) and thereafter cooled, stirred with water and extracted with ether. The organic extract was washed with water and a saturated sodium chloride solution, dried (anhy. $MgSO_4$), filtered and evaporated to 12 g oil. This oil was purified by flash chromatography (silica, 50% ethyl acetate in dichloromethane) to yield 5.5 g oil. This oil was converted to the dihydrochloride salt in methanol-ether to yield 5.5 g solid, mp 168-170°. An analytical sample was obtained by recrystallizing 3.2 g from ethanol-ether to yield 3.0 g crystals, mp 168-170°.

ANALYSIS:

| | | | |
|---|---|---|---|
| Calculated for $C_{18}H_{23}N_3O \bullet 2HCl$: | 58.38%C | 6.80%H | 11.35%N |
| Found: | 57.95%C | 6.83%H | 11.21%N |

## EXAMPLE 15

**N-[3-[1-(Phenylmethylamino)ethyl]-4-pyridinyl]-2,2-dimethylpropanamide dihydrochloride**

A solution of N-(3-acetyl-4-pyridinyl)-2,2-dimethylpropanamide (8 g), benzylamine (12 g) and p-toluenesulfonic acid monohydrate (0.2 g) in 200 ml xylene was stirred at reflux with removal of water. After eighteen hours the solution was cooled and evaporated and the product was purified by flash chromatography (silica, 50% ethyl acetate in dichloromethane) to yield 9.4 g solid, mp 112-115°. The IR ($CHCl_3$), NMR ($CDCl_3$) and mass spectra ($MH^+$ =310) were consistent with the structure of the imine intermediate. A solution of the imine (9.4 g) in 100 ml isopropanol and 25 ml methanol was stirred for two hours at 80° with sodium borohydride (2.3 g), and thereafter cooled, stirred with water and exacted with ethyl acetate. The organic extract was washed with water and a saturated sodium chloride solution, dried (anhy. $MgSO_4$), filtered and evaporated to 9 g oil. This oil was purified by flash chromatography (silica, 30% ethyl acetate in dichloromethane) to yield 6.3 g oil. A 2.3 g portion was converted to the dihydrochloride salt in methanol-ether to yield 2.3 g solid, mp 205°. This was recrystallized from methanol-ether to yield 2.2 g crystals, mp 207-209°.

ANALYSIS:

| | | | |
|---|---|---|---|
| Calculated for $C_{19}H_{27}Cl_2N_3O$: | 59.37%C | 7.08%H | 10.94%N |
| Found: | 59.20%C | 6.92%H | 10.89%N |

## EXAMPLE 16

**3-(Butylamino)methyl-4-pyridinamine dihydrochloride**

A solution prepared from N-[3-[(butylamino)methyl]-4-pyridinyl]-2,2-dimethyl-propanamide (7 g) in 150 ml methanol and 15 ml 10% aqueous sodium hydroxide was stirred at reflux for 16 hours and thereafter cooled and evaporated. The product was purified by flash chromatography (silica, 25% methanol in dichloromethane) to yield 4.5 g oil. This oil was converted to the dihydrochloride salt in methanol-ether to yield 3.7 g solid, mp 258-260°. This solid was recrystallized from ethanol to yield 3.0 g crystals, mp 258-260°.

ANALYSIS:

| | | | |
|---|---|---|---|
| Calculated for $C_{10}H_{17}N_3 \bullet 2\ HCl$: | 47.62%C | 7.59%H | 16.67%N |
| Found: | 47.53%C | 7.50%H | 16.44%N |

## EXAMPLE 17

**3-[(Phenylmethylamino)methyl]-4-pyridinamine dihydrochloride**

A solution prepared from N-[3[(phenylmethylamino)methyl]-4-pyridinyl]-2,2-dimethylpropanamide (8 g) in 200 ml methanol and 20 ml 10% aqueous sodium hydroxide was stirred at reflux for 12 hours, and thereafter cooled, stirred with water and extracted with ethyl acetate. The organic extract was washed with water and a saturated sodium chloride solution, dried (anhy. $MgSO_4$), filtered and evaporated to 7 g oil. This oil was purified by flash chromatography (silica, 25% methanol in dichloromethane) to give 3.2 g oil. This oil was converted to the dihydrochloride salt in methanol to yield 2.5 g crystals, d 305-307°.

ANALYSIS:

| | | | |
|---|---|---|---|
| Calculated for $C_{13}H_{17}Cl_2N_3$: | 54.56%C | 5.99%H | 14.68%N |
| Found: | 54.56%C | 5.88%H | 14.60%N |

**EXAMPLE 18**

**4-Amino-α-methyl-N-(phenylmethyl)-3-pyridinemethanamine**

A solution prepared from N-[3[1-(phenylmethylamino)ethyl]-4-pyridinyl]-2,2-dimethylpropanamide (12.3 g) in 200 ml n-propanol and 25 ml 10% aqueous sodium hydroxide was stirred at reflux for sixteen hours, and thereafter cooled, stirred with water and extracted with ethyl acetate-ether. The organic extract was washed with water and saturated sodium chloride, dried (anhydrous magnesium sulfate), filtered and evaporated to 8.7 g. This oil was eluted with 20% methanol in dichloromethane through silica via flash column chromatography to yield 8 g oil. This oil was eluted with 10% methanol in dichloromethane through silica via HPLC to yield 7 g oil. Following unsuccessful attempts to purify this oil as salts the reconverted free base was again eluted with 20% methanol in dichloromethane through silica via HPLC to yield 5.5 g oil. A 3.5 g portion was converted to the dimaleate salt in methanol-ether to yield 4.5 g, d 78-80°. This salt was reconverted to the free base to afford a solid which was recrystallized from 50% ether-hexane to yield 1.9 g crystals, mp 107-109°.

ANALYSIS:

| | | | |
|---|---|---|---|
| Calculated for $C_{14}H_{17}N_3$: | 73.97%C | 7.54%H | 18.49%N |
| Found: | 74.17%C | 7.57%H | 18.40%N |

**EXAMPLE 19**

**(4-Amino-3-pyridinyl)cyclohexylmethanone oxime hydrochloride**

A solution of (4-amino-3-pyridinyl)cyclohexylmethanone (5 g) and hydroxylamine hydrochloride (10 g) in 70 ml pyridine was stirred at 80° for three hours and thereafter evaporated. The residue was stirred with water, basified with sodium carbonate and extracted with dichloromethane. The organic extract was washed with water and a saturated sodium chloride solution. The dried (anhy. $MgSO_4$) organic layer was concentrated to 9 g solid. This solid was triturated with acetonitrile to yield 4.9 g solid, mp 155-158°. Three grams were converted to the hydrochloride salt in methanol-ether to yield 2.8 g solid, mp 226-228°.

ANALYSIS:

| | | | |
|---|---|---|---|
| Calculated for $C_{12}H_{18}ClN_3O$: | 56.35%C | 7.09%H | 16.43%N |
| Found: | 56.30%C | 7.17%H | 16.07%N |

**EXAMPLE 20**

**(4-Amino-3-pyridinyl)phenylmethanone oxime hydrochloride**

A solution of (4-amino-3-pyridinyl)phenylmethanone (17 g) and hydroxylamine hydrochloride (24 g) in 150 ml pyridine was stirred at 90-95° for two hours, and thereafter cooled and evaporated. The residue was stirred with water, basified with sodium carbonate and extracted with dichloromethane. The dried organic layer was evaporated to 20 g waxy solid. This was triturated with acetonitrile to yield 13.6 g solid, mp 177-179°. The filtrate was concentrated to 4.3 g oil and thereafter eluted with 10% methanol in dichloromethane through silica via flash column chromatography to yield an additional 1.8 g product for a total yield of 15.4 g. Eight grams were again eluted with 10% methanol in dichloromethane through silica via flash column chromatography to yield 6.9 g solid, mp 178-180°. A 4.9 g portion was converted to the hydrochloride salt in methanol-ether to yield 3.3 g crystals, d 238-240°.

ANALYSIS:

| | | | |
|---|---|---|---|
| Calculated for $C_{12}H_{12}ClN_3O$: | 57.72%C | 4.84%H | 16.83%N |
| Found: | 57.72%C | 4.69%H | 16.76%N |

**EXAMPLE 21**

**(4-Amino-3-pyridinyl)-(2-fluorophenyl)-methanone oxime**

A solution of (4-amino-3-pyridinyl)-(2-fluorophenyl)-methanone (19 g) and hydroxylamine hydrochloride (31 g) in 125 ml pyridine was stirred at reflux for two hours and thereafter cooled and evaporated. The residue was stirred with water, basified with sodium bicarbonate and extracted with ethyl acetate-ether. The organic extract was washed with water and saturated sodium chloride, dried (anhy. $MgSO_4$), filtered and evaporated to 25 g oil. This oil was eluted with 10% methanol in dichloromethane through silica via flash chromatography to yield 14.6 g solid, mp 170-180°. Three grams were recrystallized twice from acetonitrile to yield 1.7 g solid, mp 212-214°.

ANALYSIS:

| | | | |
|---|---|---|---|
| Calculated for $C_{12}H_{10}FN_3O$: | 62.33%C | 4.36%H | 18.18%N |
| Found: | 62.11%C | 4.41%H | 17.99%N |

**EXAMPLE 22**

**(4-Amino-3-pyridinyl)cyclohexylmethanamine dihydrochloride**

A solution of (4-amino-3-pyridinyl)cyclohexylmethanone oxime (6 g) in 145 ml 95% ethanol was quickly treated with Raney alloy (10.7 g, 50:50 Al/Ni alloy) and then with a solution of sodium hydroxide (11.4 g) in 145 ml water. The exothermic reaction was controlled with a reflux condenser. The mixture was cooled to ambient temperature and stirred for four hours. The Raney nickel catalyst (pyrophoric) was removed by filtration and the filtrate was washed with 50% aqueous ethanol. The filtrate was concentrated to remove the ethanol and the aqueous residue was extracted with dichloromethane. The dried (anhy. $MgSO_4$) organic layer was concentrated to yield 5 g oil. This was combined with 0.6 g product obtained from a trial reduction and eluted with 20% methanol in dichloromethane through silica via flash column chromatography to yield 5.0 g oil. This oil was converted to the dihydrochloride salt in methanol and thereafter the solvent was evaporated. The residue was recrystallized from 50% methanol in acetonitrile to yield 4.2 g crystals, d 314-316°.

ANALYSIS:

| | | | |
|---|---|---|---|
| Calculated for $C_{12}H_{21}Cl_2N_3$: | 51.80%C | 7.61%H | 15.11%N |
| Found: | 51.78%C | 7.45%H | 15.03%N |

EXAMPLE 23

(4-Amino-3-pyridinyl)benzenemethanamine dihydrochloride

A solution of (4-amino-3-pyridinyl)phenylmethanone oxime (6 g) in 147 ml 95% ethanol was quickly treated with Raney alloy (11 g, 50:50 Al/Ni alloy) and then with a solution of sodium hydroxide (11.7 g) in 147 ml water. The exothermic reaction was controlled with a reflux condenser. The mixture was cooled to ambient temperature and stirred for four hours. The Raney nickel catalyst (pyrophoric) was removed by filtration and the filtrate was washed with 50% aqueous ethanol. The filtrate was concentrated to remove the ethanol and the aqueous residue was extracted with dichloromethane. The dried (anhy. $MgSO_4$) organic layer was concentrated to yield 5 g solid. This solid was eluted with 20% methanol in dichloromethane through silica via flash column chromatography to yield 4.2 g, solid, mp 108-110°. This solid was converted to the dihydrochloride salt in methanol and thereafter the solvent was evaporated. The solid residue was recrystallized from 50% methanol in acetonitrile to yield 3.7 g crystals, d 280-282°.

ANALYSIS:

| | | | |
|---|---|---|---|
| Calculated for $C_{12}H_{15}Cl_2N_3$: | 52.95%C | 5.56%H | 15.44%N |
| Found: | 52.94%C | 5.47%H | 15.35%N |

## Claims

1. A compound of the formula I

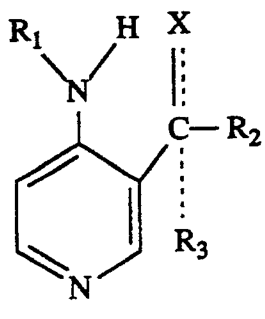

(I),

where
X is OH, O, NOH, $NH_2$, loweralkylamino, cycloalkylamino, arylloweralkylamino or arylamino;
$R_1$ is hydrogen, loweralkylcarbonyl, arylcarbonyl, arylloweralkylcarbonyl, loweralkyl or arylloweralkyl;
$R_2$ is hydrogen, loweralkyl, cycloalkyl, arylloweralkyl, aryl; and
$R_3$ when present is hydrogen, loweralkyl, cycloalkyl, arylloweralkyl or aryl with the proviso that, if X is OH or $NH_2$, $R_1$, $R_2$ and $R_3$ may not all be hydrogen; and if X = O,$R_2$ may not be hydrogen and $R_1$ may not be 2,2-dimethyl-propionyl;

or a pharmaceutically acceptable acid additon salt thereof.

2. A compound as defined in claim 1, wherein $R_1$ is hydrogen or loweralkylcarbonyl; and $R_3$ when present is hydrogen.

3. The compound as defined in claim 1, which is α-methyl-4-(2,2-dimethylpropionamido)-3-pyridinylmethanol.

4. The compound as defined in claim 1, which is α-phenyl-4-(2,2-dimethylpropionamido)-3-pyridinylmethanol.

5. The compound as defined in claim 1, which is α-cyclohexyl-4-amino-3-pyridinylmethanol.

6. The compound as defined in claim 1, which is α-phenyl-4-amino-3-pyridinylmethanol.

7. The compound as defined in claim 1, which is α-(2-fluorophenyl)-4-amino-3-pyridinylmethanol.

8. The compound as defined in claim 1, which is (4-amino-3-pyridinyl)cyclohexylmethanone.

9. The compound as defined in claim 1, which is 3-(butylamino)methyl-4-pyridinamine.

10. The compound as defined in claim 1, which is (4-amino-3-pyridinyl)-cyclohexylmethanone oxime.

11. The compound as defined in claim 1, which is (4-amino-3-pyridinyl)cyclohexylmethanamine.

12. A pharmaceutical composition which comprises a compound as defined in claim 1 as the active ingredient and a suitable carrier therefor.

13. Use of a compound as defined in claim 1 for the preparation of a medicament for the treatment of various memory dysfunctions characterized by a cholinergic deficit such as Alzheimer's disease.

14. Use of a compound as defined in claim 1 for the prepration of a medicament having analgetic activity.

15. Use of a compound as defined in claim 1 for the preparation of a medicament having a topical antiinflammatory activity for the treatment of various dermatoses.

16. A method for the preparation of a compound as defined in claim 1, which comprises
a) reacting a compound of the formula IIa

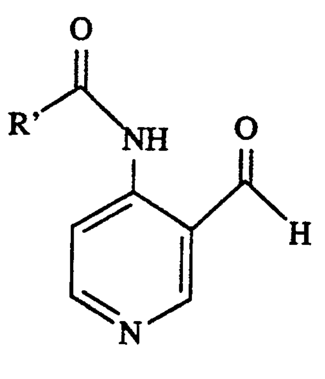

(II a),

wherein R' is loweralkyl, aryl or arylloweralkyl, with a Grignard reagent of the formula $R_2MgBr$, where $R_2$ is as defined above except that $R_2$ may not be ortho-fluorophenyl, to afford a compound of the formula I a

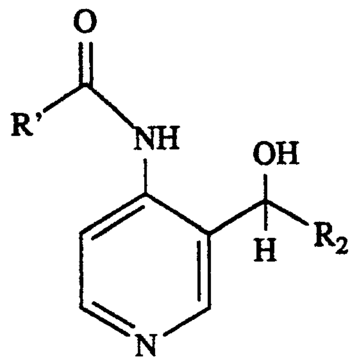

(I a)

or

b) reacting a compound of the formula

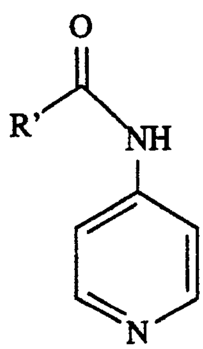

(II b),

wherein R' is as defined above with an alkyl metal and reacting the resulting dianion with an aldehyde of the formula $R_2CHO$ where $R_2$ is as defined above including that $R_2$ denotes ortho-fluoro-phenyl, to afford a compound of the formula Ia above, or

c) optionally hydrolyzing a compound of the formula Ia as obtained in step a) or b) above to afford a compound of the formula I b,

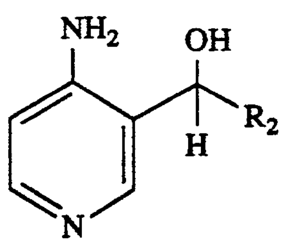

(I b),

wherein $R_2$ is as defined above, or

d) oxidizing a compound of the formula Ia as obtained in step a) or b) above to obtain a compound of the formula I c,

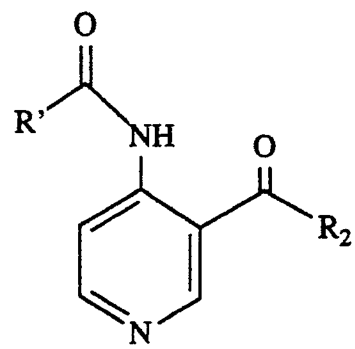

(I c),

wherein R' and $R_2$ are as defined above, or

e) hydrolyzing a compound of the formula Ic as obtained in step d) above to afford a compound of formula I d,

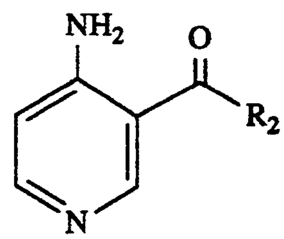

(I d),

wherein $R_2$ is as defined above, or

f) optionally reacting a compound of the formula Id as obtained in step e) above with an acid chloride of the formula $R_1$'COCl where $R_1$' is loweralkyl, arylloweralkyl or aryl to afford a compound of formula I e,

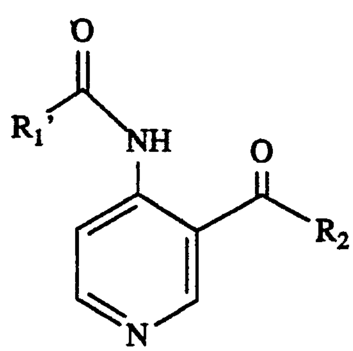

(I e)

wherein $R_1$' and $R_2$ are as defined above, or

g) reacting a compound of formula Ic as obtained in step d) above with a primary amine of the formula $H_2N-R_4$, where $R_4$ is loweralkyl, cycloalkyl, arylloweralkyl or aryl, and hydrogenating the resultant imine of formula I f,

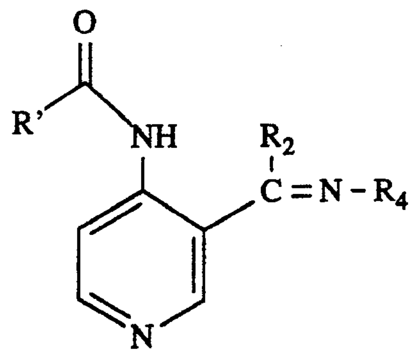

(I f),

wherein R', $R_2$ and $R_4$ are as defined above, to obtain a compound of the formula I g, wherein $R_4$, $R_2$ and R' are as defined above,

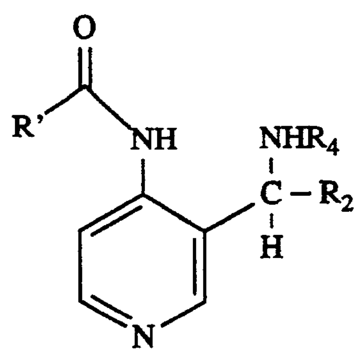

(I g),

or

h) reacting the imine of formula If) as obtained in step g) above with a Grignard reagent of the formula $R_3MgBr$ or $R_3MgCl$ ($R_3 \neq H$) to afford a compound of formula I h

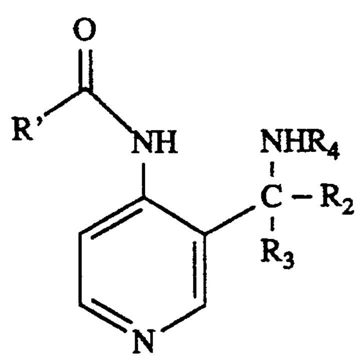

(I h),

wherein R', $R_2$, $R_4$ and $R_3$ are as defined above, except that $R_3$ may not be hydrogen, or

i) optionally hydrolyzing a compound of the formula I h or Ig as obtained in step h) or in the second reaction of step g) above to afford a compound of the formula Ij

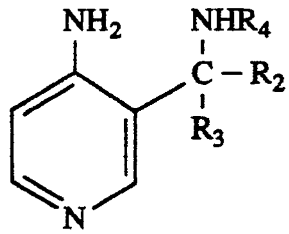

(I j),

wherein $R_2$, $R_3$ and $R_4$ are as defined above,

j) reacting a compound of formula Ie as obtained in step f) above with a primary amine of the formula $H_2N$-$R_4$, wherein $R_4$ has the above-mentioned meaning, and hydrogenating the resultant imine of formula I k

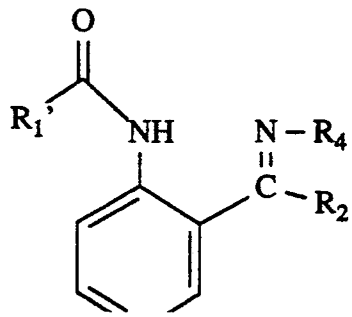

(I k),

wherein $R_1$', $R_2$ and $R_4$ are as defined above, to obtain a compound of the formula I l

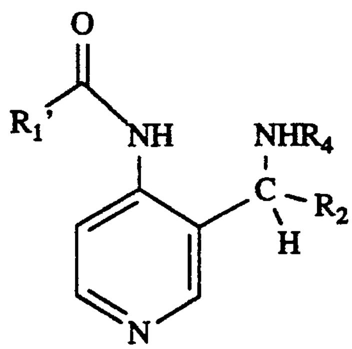

(I l),

wherein $R_4$, $R_2$ and $R_1$ are as defined above, or

k) optionally reducing the compound of formula I l as obtained in step j) above to afford a compound of formula I m

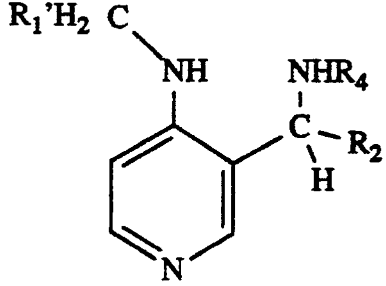

(I m),

wherein $R_1$', $R_4$ and $R_2$ are as defined above, or

l) reacting a compound of formula I d as obtained in step e) above with hydroxylamine to afford an oxime compound of formula I n

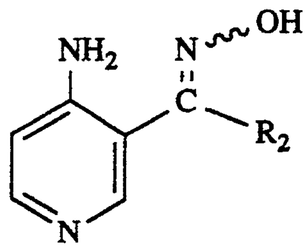

(I n),

wherein $R_2$ is as defined above, or

m) optionally hydrogenating a compound of formula I n as obtained in step 1) above to afford a compound of the formula I p

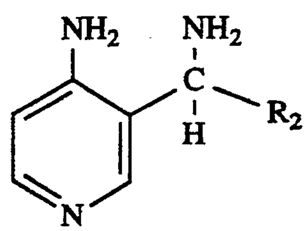

(I p),

wherein $R_2$ is as defined above.

Claims for the following Contracting States : ES, GR:

1. A process for the preparation of a compound of the formula I

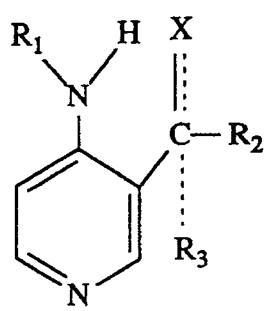

(I),

where

X is OH, O, NOH, $NH_2$, loweralkylamino, cycloalkylamino, arylloweralkylamino or arylamino;

$R_1$ is hydrogen, loweralkylcarbonyl, arylcarbonyl, arylloweralkylcarbonyl, loweralkyl or arylloweralkyl;

$R_2$ is hydrogen, loweralkyl, cycloalkyl, arylloweralkyl, aryl; and

$R_3$ when present is hydrogen, loweralkyl, cycloalkyl, arylloweralkyl or aryl with the proviso that, if X is OH or $NH_2$, $R_1$, $R_2$ and $R_3$ may not all be hydrogen; and if X = O, $R_2$ may not be hydrogen and $R_1$ may not be 2,2-dimethyl-propionyl;

or a pharmaceutically acceptable acid additon salt thereof, which comprises

a) reacting a compound of the formula II a

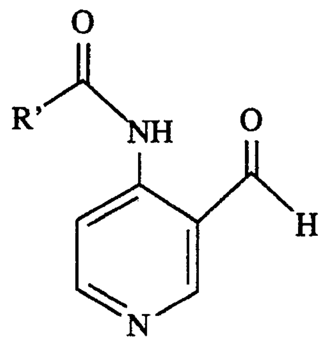

(II a),

wherein R' is loweralkyl, aryl or arylloweralkyl, with a Grignard reagent of the formula $R_2MgB_6$, where $R_2$ is as defined above except that $R_2$ may not be ortho-fluorophenyl, to afford a compound of the formula I a

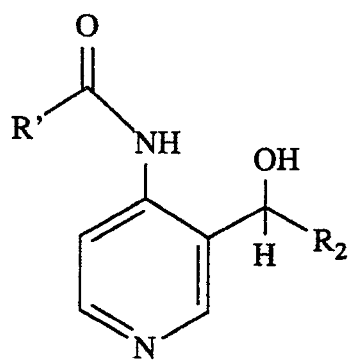

(I a)

or

b) reacting a compound of the formula

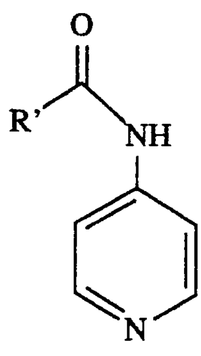

(II b),

wherein R' is as defined above with an alkyl metal and reacting the resulting dianion with an aldehyde of the formula $R_2CHO$ where $R_2$ is as defined above including that $R_2$ denotes ortho-fluoro-phenyl, to afford a compound of the formula I a above, or

c) optionally hydrolyzing a compound of the formula I a as obtained in step a) or b) above to afford a compound of the formula I b,

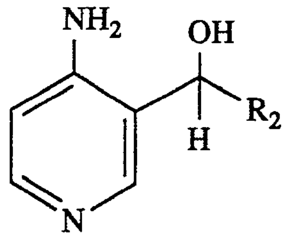

(I b),

wherein $R_2$ is as defined above, or

d) oxidizing a compound of the formula I a as obtained in step a) or b) above to obtain a compound of the formula I c,

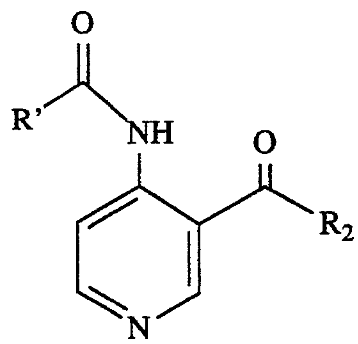

(I c),

wherein R' and $R_2$ are as defined above, or

e) hydrolyzing a compound of the formula I c as obtained in step d) above to afford a compound of formula I d,

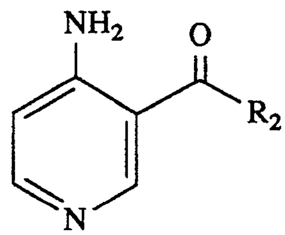

(I d),

wherein $R_2$ is as defined above, or

f) optionally reacting a compound of the formula I d as obtained in step e) above with an acid chloride of the formula $R_1$'COCl where $R_1$' is loweralkyl, arylloweralkyl or aryl to afford a compound of formula I e,

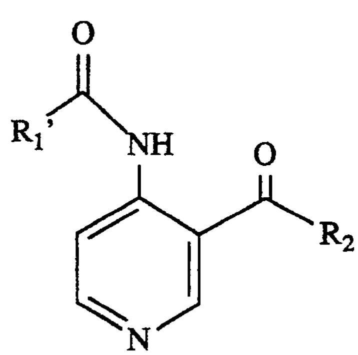

(I e),

wherein $R_1$' and $R_2$ are as defined above, or

g) reacting a compound of formula I c as obtained in step d) above with a primary amine of the formula $H_2N-R_4$, where $R_4$ is loweralkyl, cycloalkyl, arylloweralkyl or aryl, and hydrogenating the resultant imine of formula I f,

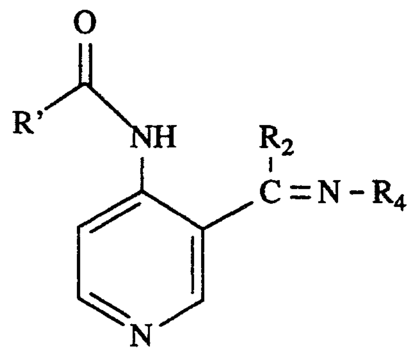
(I f),

wherein R', $R_2$ and $R_4$ are as defined above, to obtain a compound of the formula I g, wherein $R_4$, $R_2$ and R' are as defined above,

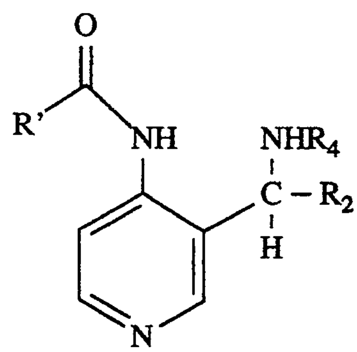
(I g),

or

h) reacting the imine of formula I f) as obtained in step g) above with a Grignard reagent of the formula $R_3MgBr$ or $R_3MgCl$ ($R_3 \neq H$) to afford a compound of formula I h

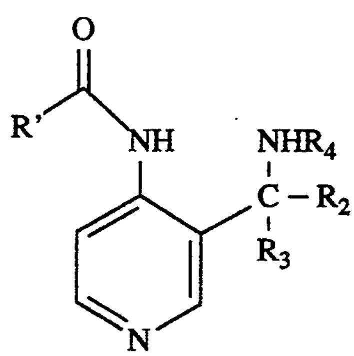
(I h),

wherein R', $R_2$, $R_4$ and $R_3$ are as defined above, except that $R_3$ may not be hydrogen, or

i) optionally hydrolyzing a compound of the formula I h or I g as obtained in step h) or in the second reaction of step g) above to afford a compound of the formula I j

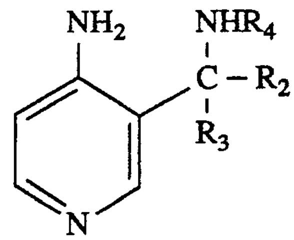

(I j),

wherein $R_2$, $R_3$ and $R_4$ are as defined above,

j) reacting a compound of formula I e as obtained in step f) above with a primary amine of the formula $H_2N$-$R_4$, wherein $R_4$ has the above-mentioned meaning, and hydrogenating the resultant imine of formula I k

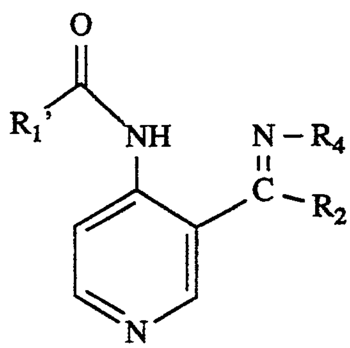

(I k),

wherein $R_1'$, $R_2$ and $R_4$ are as defined above, to obtain a compound of the formula I l

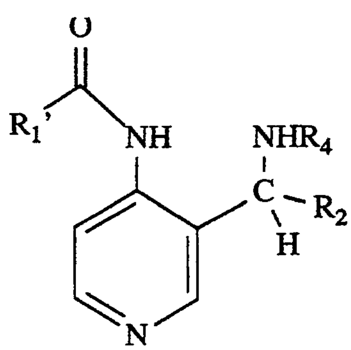

(I l),

wherein $R_4$, $R_2$ and $R_1$ are as defined above, or

k) optionally reducing the compound of formula I l as obtained in step j) above to afford a compound of formula I m

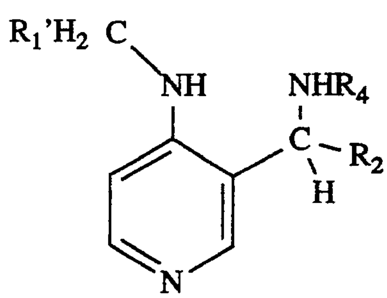

(I m),

wherein $R_1'$, $R_4$ and $R_2$ are as defined above, or

l) reacting a compound of formula I d as obtained in step e) above with hydroxylamine to afford an oxime compound of formula I n

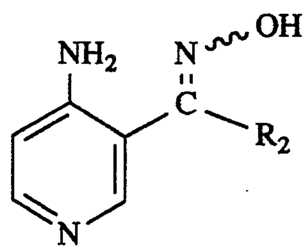

(I n),

wherein $R_2$ is as defined above, or

m) optionally hydrogenating a compound of formula I n as obtained in step 1) above to afford a compound of the formula I p

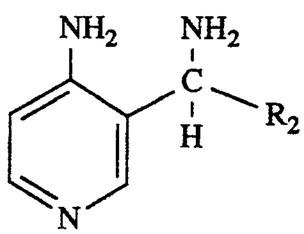

(I p),

wherein $R_2$ is as defined above.

2. A process as defined in claim 1, wherein $R_1$ is hydrogen or loweralkylcarbonyl; and $R_3$ when present is hydrogen.

3. The process as defined in claim 1, wherein is α-methyl-4-(2,2-dimethylpropionamido)-3-pyridinyl-methanol is prepared.

4. The process as defined in claim 1, wherein α-phenyl-4-(2,2-dimethylpropionamido)-3-pyridinylmethanol is prepared.

5. The process as defined in claim 1, wherein α-cyclohexyl-4-amino-3-pyridinylmethanol is prepared.

6. The process as defined in claim 1, wherein α-phenyl-4-amino-3-pyridinyl-methanol is prepared.

7. The process as defined in claim 1, wherein α-(2-fluorophenyl)-4-amino-3-pyridinylmethanol is prepared.

8. The process as defined in claim 1, wherein (4-amino-3-pyridinyl)cyclohexylmethanone is prepared.

9. The process as defined in claim 1, wherein 3-(butylamino)methyl-4-pyridinamine is prepared.

10. The process as defined in claim 1, wherein (4-amino-3-pyridinyl)-cyclohexylmethanone oxime is prepared.

11. The process as defined in claim 1, wherein (4-amino-3-pyridinyl)cyclohexylmethanamine is prepared.

12. Use of a compound as defined in claim 1 for the preparation of a medicament for the treatment of various memory dysfunctions characterized by a cholinergic deficit such as Alzheimer's disease.

13. Use of a compound as defined in claim 1 for the prepration of a medicament having analgetic activity.

**14.** Use of a compound as defined in claim 1 for the preparation of a medicament having a topical antiinflammatory activity for the treatment of various dermatoses.